# EUROPEAN PATENT SPECIFICATION

(11) **EP 2 487 150 B1**
(45) Date of publication and mention of the grant of the patent: **28.12.2016**
(21) Application number: 10822148.2
(22) Date of filing: 08.10.2010
(51) Int. Cl.: C07C 45/66, C07C 49/395

(54) **PROCESS FOR PRODUCING A LACTONE**
VERFAHREN ZUR HERSTELLUNG EINES LACTONS
PROCÉDÉ DE PRODUCTION D'UN LACTONE

(30) Priority: 09.10.2009 JP 2009235127
(43) Date of publication of application: 15.08.2012
(73) Proprietor: Kao Corporation, Chuo-Ku Tokyo 103-8210 (JP)
(72) Inventor: MATSUMOTO, Kunshi, Wakayama-shi Wakayama 640-8580 (JP); NAGASAWA, Atsushi, Wakayama-shi Wakayama 640-8580 (JP); ATAKA, Yoshiharu, Wakayama-shi Wakayama 640-8580 (JP)
(74) Representative: Hoffmann Eitle
(86) International application number: PCT/JP2010/067799
(87) International publication number: WO 2011/043478

(56) References cited:
- EP-A1- 2 266 943
- GB-A- 745 946
- JP-A- 61 005 038
- JP-A- 2004 203 844
- JP-A- 2009 298 771
- MEKHTIEV, S.D. ET AL.: 'Synthesis of 2-alkylcyclopentanones' AZERBAIDZHANSKII KHIMICHESKII ZHURNAL 1975, pages 34 - 38, XP008153538
- XU, J. ET AL.: 'Synthesis of 5-decalactone and delta-dodecalactone' JINGXI YU ZHUANYONG HUAXUEPIN vol. 12, no. 21, 2004, pages 12 - 14, XP008153558
- MAY, W.A. ET AL.: 'Synthesis of some unsaturated lactones and their relationship to deep-fat fried flavor' JOURNAL OF FOOD SCIENCE vol. 43, no. 4, 1978, pages 1248 - 1252, XP008153477
- STUKANOVA, L.N. ET AL.: 'Synthesis and properties of bicyclopentyls' NEFTEKHIMIYA vol. 4, no. 4, 1964, pages 521 - 529, XP008153536

## Description

### FIELD OF THE INVENTION

The present invention relates to a process for producing lactones via 2-alkylcycloalkanones.

### BACKGROUND OF THE INVENTION

The 2-alkylcycloalkanones are useful substances as an intermediate product for synthesis of physiologically active substances or perfume materials. Hitherto, the 2-alkylcycloalkanones have been produced by first subjecting 2-(1-hydroxyalkyl)-cycloalkanones to dehydration reaction to synthesize 2-(alkylidene)-cycloalkanones, and then subjecting the 2-(alkylidene)-cycloalkanones to hydrogenation reaction.

It is generally known that the above dehydration reaction is carried out in the presence of an acid. For example, Patent Documents 1 and 2 disclose the dehydration reaction using oxalic acid or phosphoric acid. Also, Patent Document 3 discloses the dehydration reaction using a solid acid.

In addition, as the hydrogenation reaction subsequent to the dehydration reaction, there has been reported an example of the reaction using a metal catalyst. For example, Non-Patent Document 1 discloses the hydrogenation reaction using a platinum group metal catalyst.

On the other hand, there is known the method in which the dehydration reaction and hydrogenation reaction are carried out only in one step. For example, Patent Document 4 discloses the method in which diacetone alcohol is contacted with a hydrogen gas under pressure in the presence of an acid catalyst and a reducing catalyst to carry out the dehydration reaction and hydrogenation reaction thereof in one step and thereby directly convert the diacetone alcohol to methyl isobutyl ketone.

Patent Document 1: JP 56-147740A
Patent Document 2: JP 2004-217620A
Patent Document 3: JP 2004-203844A
Patent Document 4: JP 61-5038A

Non-Patent Document 1: Warren, "Organic Chemistry", Tokyo Kagaku Dojin K.K., p. 626

GB 745 946 describes a process for the production of saturated ketones which comprises hydrogenating a ketol in the presence of a hydrogenation catalyst and a free acid which remains a free-acid in the presence of hydrogen at elevated temperature.

EP 2 266 943 A1 represents a prior right under Article 54(3) EPC and relates to a process for producing a 2-alkyl-2-cycloalken-1-one, which includes the step of subjecting a 2-(1-hydroxyalkyl)cycloalkan-1-one to dehydration and isomerization in the co-existence of an acid and a platinum group metal catalyst, and to a process for producing an alkyl(3-oxo-alkylcycloalkyl)acetate which is useful as a perfume material.

May et al., Journal of Food Science 43(4), 1248-1252 (1978) describe the synthesis of seven unsaturated lactones, i.e., the lactones of 4-hydroxy-nonenoic acid, with the double bond at the 2 and 3 position, respectively, 4-hydroxy-octenoic acid with the double bond at the 2 and 3 position, respectively, 5-hydroxy-4-nonenoic acid, 5-hydroxy-2-octenoic acid, and 5-hydroxy-2-undecenoic acid. Their chemical structures were confirmed by their infrared and mass spectra.

Xu et al., Jingxi Yu Zhuanyong Huaxuepin (Fine and Specialty Chemicals), Vol. 12, No. 21, 12-14 (2004) discloses a synthesis of 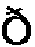 -decalactone and 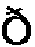 -dodecalactone via 2-(1-hydroxyalkyl)cycloalkanones and 2-alkylcycloalkanones as intermediates.

### SUMMARY OF THE INVENTION

In the methods in which 2-(1-hydroxyalkyl)-cycloalkanones are subjected to dehydration reaction and then hydrogenation reaction, the dehydration reaction may be carried out in the presence of an acid as described in Patent Documents 1 to 3, and then the hydrogenation reaction may be carried out in the presence of a metal catalyst as described in Non-Patent Document 1. However, these methods not only tend to be deteriorated in productivity owing to increase in number of steps, but also tend to be unsatisfactory in yield of the aimed product.

On the other hand, in the method as described in Patent Document 4 in which the dehydration reaction and hydrogenation reaction are carried out in one step, since the reaction rate varies depending upon a hydrogen pressure upon the reaction, the reaction tends to proceed very slowly under a low pressure condition. Therefore, the reaction is usually carried out under a relatively high pressure condition such as under a pressure of about 1.08 MPa (absolute pressure). However, in the case where the above method is applied to 2-(1-hydroxyalkyl)-cycloalkanones, a compound formed by reducing a carbonyl group of 2-alkylcycloalkanones as the aimed product is produced as a by-product. The resulting by-product tends to be hardly separated from 2-alkylcycloalkanones as the aimed product when subjected to a purification step.

The present invention relates to a process for producing lactones as a useful perfume material for cosmetics, flavors, etc., in which 2-alkylcycloalkanones are produced with high yield and high purity, and further subjected to an oxidation reaction.

The present inventors have found that when subjecting 2-(1-hydroxyalkyl)-cycloalkanones to dehydration and hydrogenation reaction in a flow of a hydrogen gas under a low pressure in the presence of an acid and a platinum group metal catalyst, it is possible to synthesize 2-alkylcycloalkanones with a high yield and a high purity.

Thus, the present invention relates to [1] a process for producing a lactone via a 2-alkylcycloalkanone represented by the following general formula (2), including the step of subjecting a 2-(1-hydroxyalkyl)-cycloalkanone represented by the following general formula (1) to dehydration and hydrogenation reaction in a flow of a hydrogen gas under a pressure of from 20 to 200 kPa (absolute pressure) in the presence of an acid and a platinum group metal catalyst; and then subjecting the 2-alkylcycloalkanone to oxidation reaction using a percarboxylic acid. 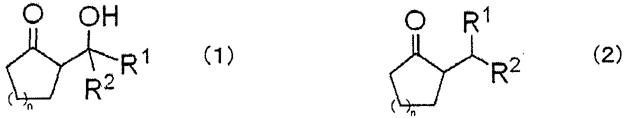 wherein n is an integer of 1 or 2; and R¹ and R² are each independently a hydrogen atom or an alkyl group having 1 to 8 carbon atoms with the proviso that R¹ and R² may form a cyclopentane ring or a cyclohexane ring through a carbon atom adjacent thereto.

### EFFECT OF THE INVENTION

In accordance with the present invention, there is provided a process for producing lactones as a useful perfume material for cosmetics, flavors, etc., efficiently and at low costs.

### DETAILED DESCRIPTION OF THE INVENTION

The process for producing a 2-alkylcycloalkanone represented by the following general formula (2) (hereinafter occasionally referred to merely as a "compound (2)") according to the first step of the process of the invention is characterized in that a 2-(1-hydroxyalkyl)-cycloalkanone represented by the following general formula (1) (hereinafter occasionally referred to merely as a "compound (1)") is subjected to dehydration and hydrogenation reaction in a flow of a hydrogen gas under a pressure of from 20 to 200 kPa (absolute pressure) in the presence of an acid and a platinum group metal catalyst. 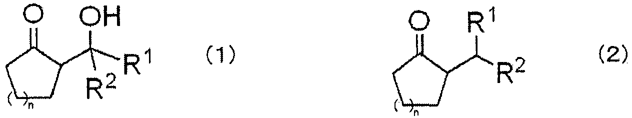 wherein n is an integer of 1 or 2; and R¹ and R² are each independently a hydrogen atom or an alkyl group having 1 to 8 carbon atoms with the proviso that R¹ and R² may form a cyclopentane ring or a cyclohexane ring through a carbon atom adjacent thereto.

### [Compounds (1) and (2)]

In the process for producing the compound (2) as the aimed product according to the present invention, the compound (1) is used as a raw material thereof.

In the above general formulae (1) and (2), R¹ and R² are each independently a hydrogen atom or an alkyl group having 1 to 8 carbon atoms with the proviso that R¹ and R² may form a cyclopentane ring or a cyclohexane ring through a carbon atom adjacent thereto. R¹ and R² are each preferably a hydrogen atom or a linear or branched alkyl group, and more preferably a hydrogen atom or a linear alkyl group.

Examples of the alkyl group as R¹ and R² include a methyl group, an ethyl group, an n-propyl group, an isopropyl group, various butyl groups, various pentyl groups, various hexyl groups, various heptyl groups and various octyl groups. The term "various" as used herein means "linear" or "branched".

The clause "R¹ and R² may form a cyclopentane ring or a cyclohexane ring through a carbon atom adjacent thereto" as used herein means that "R¹ may be bonded to R² through the carbon atom, or R² may be bonded to R¹ through the carbon atom, to form a 5-membered ring or a 6-membered ring". Meanwhile, hydrogen atoms bonded to carbon atoms constituting the 5-membered ring or 6-membered ring may be substituted with a hydrocarbon group such as an alkyl group or alkenyl group having 1 to 5 carbon atoms.

Specific examples of the compound (1) include 2-(1-hydroxypropyl)-cyclopentanone, 2-(1-hydroxybutyl)-cyclopentanone, 2-(1-hydroxypentyl)-cyclopentanone, 2-(1-hydroxyhexyl)-cyclopentanone, 2-(1-hydroxyheptyl)-cyclopentanone, 2-(1-hydroxy-1-methylbutyl)-cyclopentanone, 2-(1-hydroxy-2-methylbutyl)-cyclopentanone, 2-(1-hydroxycyclopentyl)-cyclopentanone, 2-(1-hydroxycyclohexyl)-cyclopentanone, 2-(1-hydroxypropyl)-cyclohexanone, 2-(1-hydroxybutyl)-cyclohexanone, 2-(1-hydroxypentyl)-cyclohexanone, 2-(1-hydroxyhexyl)-cyclohexanone, 2-(1-hydroxyheptyl)-cyclohexanone, 2-(1-hydroxy-1-methylbutyl)-cyclohexanone, 2-(1-hydroxy-2-methylbutyl)-cyclohexanone, 2-(1-hydroxycyclopentyl)-cyclohexanone and 2-(1-hydroxycyclohexyl)-cyclohexanone.

Among these compounds (1), preferred are 2-(1-hydroxypropyl)-cyclopentanone, 2-(1-hydroxybutyl)-cyclopentanone, 2-(1-hydroxypentyl)-cyclopentanone and 2-(1-hydroxyhexyl)-cyclopentanone, and especially preferred is 2-(1-hydroxypentyl)-cyclopentanone.

Specific examples of the compound (2) include 2-propylcyclopentanone, 2-butylcyclopentanone, 2-pentylcyclopentanone, 2-hexylcyclopentanone, 2-heptylcyclopentanone, 2-(1-methylbutyl)-cyclopentanone, 2-(2-methylbutyl)-cyclopentanone, 2-cyclopentylcyclopentanone, 2-cyclohexylcyclopentanone, 2-propylcyclohexanone, 2-butylcyclohexanone, 2-pentylcyclohexanone, 2-hexylcyclohexanone, 2-heptylcyclohexanone, 2-(1-methylbutyl)-cyclohexanone, 2-(2-methylbutyl)-cyclohexanone, 2-cyclopentylcyclohexanone and 2-cyclohexylcyclohexanone.

Among these compounds (2), preferred are 2-propylcyclopentanone, 2-butylcyclopentanone, 2-pentylcyclopentanone and 2-hexylcyclopentanone, and especially preferred is 2-pentylcyclopentanone.

### [Method for Producing Compound (1)]

The compound (1) may be produced by known methods. For example, the compound (1) may be produced by reacting a cycloalkanone having 5 or 6 carbon atoms with an aldehyde or a ketone represented by the following formula (3).

In the present invention, the compound (1) produced by the above method may be used as such without being purified, but may also be purified before use by distillation, etc. 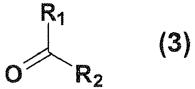 wherein R¹ and R² are the same as defined above.

### [Process for Producing Compound (2)]

In the present invention, the compound (2) as the aimed product is produced by subjecting the compound (1) to dehydration and hydrogenation reaction in a flow of a hydrogen gas under a pressure of from 20 to 200 kPa (absolute pressure) in the presence of an acid and a platinum group metal catalyst.

### <Acid>

In the present invention, as the acid, there may be used an inorganic acid, an organic acid and a solid acid, etc.

### (Inorganic Acid and Organic Acid)

The inorganic acid and the organic acid used in the present invention may be ordinary inorganic and organic acids. Specific examples of the acid include inorganic acids such as hydrochloric acid, sulfuric acid, phosphoric acid, orthophosphoric acid, and condensed phosphoric acids such as metaphosphoric acid, pyrophosphoric acid and tripolyphosphoric acid; and organic acids such as acetic acid, oxalic acid, citric acid, maleic acid, fumaric acid, malic acid, p-toluenesulfonic acid and methane-sulfonic acid.

Among these acids, from the viewpoints of suppressing a polymerization reaction as a side reaction and reducing a burden of costs for facilities, preferred are those acids having a first acid dissociation constant (pKa) of 0 or more and preferably 0.5 or more as measured at 25°C. More specifically, as the preferred acids, there may be mentioned phosphoric acid (first pKa: 2.15), condensed phosphoric acids such as pyrophosphoric acid (first pKa: 0.8) and tripolyphosphoric acid, and organic acids including, e.g., aliphatic monocarboxylic acids having 2 to 8 carbon atoms such as acetic acid (pKa: 4.56), lactic acid (pKa: 3.66), valeric acid (pKa: 4.64) and octanoic acid (pKa: 4.89), aliphatic dicarboxylic or tricarboxylic acids having 2 to 8 carbon atoms such as oxalic acid (first pKa: 1.04), citric acid (first pKa: 2.87), maleic acid (first pKa: 1.75), fumaric acid (first pKa: 2.85), malic acid (first pKa: 3.24) and adipic acid (first pKa: 4.26), and aromatic carboxylic acids such as benzoic acid (pKa: 4.20) and phthalic acid (first pKa: 2.95).

In addition, from the viewpoint of boiling point, more preferred are phosphoric acid, condensed phosphoric acids such as pyrophosphoric acid and tripolyphosphoric acid, and organic acids such as oxalic acid, citric acid, maleic acid, fumaric acid and malic acid, and especially preferred are phosphoric acid and oxalic acid.

The acid dissociation constant (pKa) as used in the present invention is described, for example, in "Chemical Handbook", The Chemical Society of Japan (revised 3rd edition, published by Maruzen K.K. on June 25, 1984).

These acids may be supported on a carrier. Examples of the carrier include silica and activated carbon. Among these carriers, preferred is activated carbon. The acid may be supported on the carrier by the method in which the carrier is impregnated with the acid, or the method in which phosphoric acid is added and penetrated into a wood material (such as sawdust and wood chips) at a high temperature while suppressing generation of tar to erode fibers of the wood material, and then the thus treated wood material is subjected to carbonization reaction at a temperature of from 500 to 700°C under air-free conditions to obtain a phosphoric acid-supporting activated carbon (also referred to as a "phosphoric acid-activated carbon"). Among these acid-supporting carriers, the phosphoric acid-supporting activated carbon is especially preferred.

Such an acid as supported on the carrier can be handled in the same manner as the below-mentioned solid acid, and is readily separated and removed from the reaction mixture.

These acids may be used alone or in combination of any two or more thereof.

### (Solid Acid)

As the solid acid, there may be used conventionally known solid acids. Specific examples of the solid acid include inorganic metal solids such as activated alumina, zirconia sulfate, metal phosphates, aluminum dihydrogen tripolyphosphate and titanium oxide; cation exchange resins; and silica-titania composite oxide, silica-calcium oxide composite oxide, silica-magnesia composite oxide and zeolite.

The solid acid used in the present invention preferably satisfies such a condition in which an amount (mmol/g) of acid sites of the solid acid from which ammonia (NH₃) is desorbed at a temperature of from 100 to 250°C is larger than an amount (mmol/g) of acid sites of the solid acid from which NH₃ is desorbed at a temperature higher than 250°C, as measured by an ammonia temperature-programmed desorption (TPD) method. The amount of acid sites of the solid acid from which NH₃ is desorbed at a temperature of from 100 to 250°C is preferably 0.3 mmol/g or more, whereas the amount of acid sites of the solid acid from which NH₃ is desorbed at a temperature higher than 250°C is preferably less than 0.3 mmol/g.

In the above TPD method, the amount of acid sites of the solid acid is determined as a relative amount based on an amount (0.99 mmol/g) of acid sites of zeolite "JRC-Z5-25H" as a reference catalyst prescribed by The Catalysis Society of Japan which is measured at a high peak (peak on a high-temperature side among two kinds of peaks observed). The peak is detected by quantitative determination of ammonia using a fragment m/e=16 of the ammonia in a mass spectrum.

The TPD (ammonia temperature-programmed desorption) may be measured by an ordinary method generally used therefor. For example, the TPD measurement may be carried out after sequentially subjecting the acid to a pretreatment, an NH₃ absorption treatment and a vacuum treatment under the following conditions.
Pretreatment: Temperature is raised up to 200°C in helium over 20 min, and maintained at the same temperature for 1 h;
NH₃ absorption treatment: NH₃ is absorbed at 50°C under 2.7 kPa for 10 min;
Vacuum treatment: Treated at 50°C for 4 h; and
TPD measurement: While flowing a helium gas at a rate of 50 mL/min, temperature is raised up to 600°C at a rate of 5°C/min.

As the solid acid having such an acid site distribution, there are preferably used, for example, those solid acids having at least one of the following structure (A), structure (B) and metal atom (C). Among these solid acids, preferred are the solid acids having the structure (A) and the metal atom (C), the solid acids having the structure (B) and the metal atom (C), and the solid acids having the structure (A), the structure (B) and the metal atom (C).
- Structure (A): Structure in which a hydrogen atom is removed from at least one OH group contained in an inorganic phosphoric acid.
- Structure (B): Structure in which a hydrogen atom is removed from at least one OH group contained in an organic phosphoric acid represented by the following general formula (4) or (5).
- Metal atom (C): One or more metal atoms selected from the group consisting of aluminum, gallium and iron. 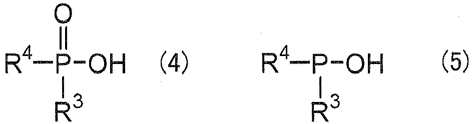
wherein R³ and R⁴ are each selected from R⁶, OR⁶, OH and H with the proviso that at least one of R³ and R⁴ is R⁶ or OR⁶ wherein R⁶ is an organic group having 1 to 22 carbon atoms.

As the structure (A), there may be mentioned the structures derived from orthophosphoric acid, condensed phosphoric acids such as metaphosphoric acid and pyrophosphoric acid, etc. Among these structures, from the viewpoint of good performance, preferred is the structure (A) derived from orthophosphoric acid.

Examples of the organic phosphoric acid represented by the general formula (4) or (5) in the structure (B) include phosphonic acid, phosphonic acid monoesters, phosphinic acid, phosphoric acid monoesters, phosphoric acid diesters, phosphorous acid monoesters and phosphorous acid diesters. Among these structures, preferred is the structure (B) derived from phosphonic acid.

R⁶ is preferably an organic group preferably having 1 to 15 carbon atoms and more preferably 1 to 8 carbon atoms. Examples of the organic group as R⁶ include alkyl groups such as a methyl group, an ethyl group, a propyl group, an isopropyl group, various butyl groups, various pentyl groups, various hexyl groups and various octyl groups; and aryl groups such as a phenyl group and a 3-methyl phenyl group.

As the metal atom (C), aluminum is preferred from the viewpoints of good performance and/or low costs.

Meanwhile, for the purpose of improving a selectivity and other performances, the metal atom (C) may contain a small amount of a metal atom other than aluminum, gallium and iron. Also, it is not necessarily required that a whole of the metal atom (C) contained in the catalyst is bonded to the structure (A) or (B), and only a part of the metal atom (C) may be present in the form of a metal oxide or a metal hydroxide.

The solid acid may be produced by a precipitation method, a method of impregnating a metal oxide or a metal hydroxide with an inorganic phosphoric acid and an organic phosphoric acid, a method of substituting an inorganic phosphoric acid group contained in an inorganic aluminum phosphate gel with an organic phosphoric acid group, etc. Among these methods, preferred is the precipitation method.

Upon production of the solid acid, a carrier having a large surface area may be allowed to coexist therewith to obtain a supported catalyst. Examples of the carrier include silica, alumina, silica/alumina, titania, zirconia, diatomaceous earth and activated carbon. When the carrier is used in an excessive amount, the resulting supported catalyst tends to be deteriorated in catalytic activity owing to a low content of the active component. Therefore, the content of the carrier in the catalyst is preferably 90% by mass or less.

The solid acid may be in the form of either a powder or a molded product. In addition, the solid acids may respectively have the same composition or may be used in combination of two or more kinds thereof which are different in composition from each other.

The above inorganic acids, organic acids and solid acids may be respectively used alone or in combination of any two or more thereof. When using the solid acid solely, the neutralization step may be omitted.

The amount of the acid used is preferably 0.0001% by mass or more on the basis of the compound (1) as the raw material from the viewpoint of good reactivity. On the other hand, from the viewpoints of suppressing occurrence of polymerization and thereby enhancing a yield of the aimed product, the amount of the acid used is preferably 25% by mass or less. From the above viewpoints, the amount of the acid used is more preferably from 0.001 to 12% by mass and still more preferably from 0.01 to 6% by mass.

### <Platinum Group Metal Catalyst>

The platinum group metal catalyst used in the present invention contains one or more metal components selected from the group consisting of osmium (Os), ruthenium (Ru), iridium (Ir), rhodium (Rh), platinum (Pt) and palladium (Pd) belonging to elements of Groups 8 to 10 in the 5th and 6th periods of the Periodic Table as main components. Among these metal components, from the viewpoint of catalytic activity, etc., Pt and Pd are preferred. These metal components may be used alone or in combination of any two or more thereof. The term "containing the metal components as main components" as used herein means that the metal components are preferably contained in an amount of 50 mol% or more, more preferably 70 mol% or more, still more preferably 90 mol% or more, and further still more preferably 95 mol% or more on the basis of the whole catalyst metal components.

The platinum group metal catalyst may also contain other metal components or may contain a co-catalyst only in an auxiliary amount. Examples of the other metal components include elements of Groups 4 to 11 in the 4th period of the Periodic Table such as Ti, V, Cr, Mn, Fe, Co, Ni and Cu, as well as W, Ag and Au.

The catalyst may be formed into an appropriate configuration such as a supporting type, a Raney type, a solution type, a powder type and a granule type when used in the process.

The supporting type catalyst is a catalyst of such a type in which the metal components are supported on a carrier in order to improve physical properties thereof such as durability. The supporting type catalyst may be prepared by known methods such as a precipitation method, an ion exchange method, an evaporation-to-dryness method, a spray-drying method and a kneading method. Examples of the carrier include carbon (activated carbon), alumina, silica, silica-alumina, barium sulfate and calcium carbonate. Among these carriers, preferred are carbon (activated carbon), silica, alumina and silica-alumina.

Specific examples of the palladium catalyst or platinum catalyst used as the supporting type catalyst include palladium on carbon, palladium on alumina, palladium on barium sulfate, palladium on calcium carbonate, platinum on carbon, platinum on alumina, platinum on barium sulfate and platinum on calcium carbonate. Among these supporting type catalysts, palladium on carbon and palladium on alumina are preferred because these catalysts have a high reactivity and can be readily recovered from the reaction mixture after completion of the reaction, and palladium on carbon is especially preferred from the viewpoints of a good availability, easiness of handing and a high reactivity.

The amount of the metal components supported on the carrier in the supporting type catalyst is usually from about 0.1 to about 70% by mass on the basis of a total amount of the carrier and the metal components supported thereon from the viewpoint of good catalytic activity.

The Raney type catalyst is a porous spongy metal catalyst, and may be prepared, for example, by the method described in Teruo KUBOMATSU and Shinichiro KOMATSU "Raney Catalysts", Kyoritsu-Shuppan (1971), etc.

When using the solution type catalyst, an aqueous solution containing a metal salt of an inorganic acid such as, for example, nitric acid and hydrochloric acid, or a mixed aqueous solution of various metal salts, may be added dropwise to the reaction system.

Meanwhile, as the above catalyst, there may also be used commercially available products.

The amount of the platinum group metal catalyst used in the above process may be optimized according to the type of the reaction.

In the case of the batch type reaction, from the viewpoints of a good reactivity and economy, the platinum group metal catalyst is preferably used in an amount of from 0.0002 to 3% by mass, more preferably from 0.002 to 2% by mass and still more preferably from 0.005 to 1% by mass in terms of an amount of the metal on the basis of the compound (1) as the raw material.

The acid and the platinum group metal catalyst may be respectively used in separate forms or may be used as an integrated catalyst having one configuration. For example, if the platinum group metal catalyst is supported on an acid carrier, it is not required to separately add an additional acid thereto.

The acid and the platinum group metal catalyst may also be used either in a suspended bed or a fixed bed.

The fixed bed reaction using the carrier-supported acid or the solid acid is effective for mass-production of the aimed compound because no step of separating the catalyst, etc., from the final reaction product is required.

Even in the suspended bed reaction, as far as the solid acid is used therein, the catalyst, etc., may be readily separated by filtration, etc., from the reaction solution, and the thus separated catalyst can be suitably recycled.

### <Dehydration and Hydrogenation Reaction>

The dehydration and hydrogenation reaction may be carried out using the above acid and platinum group metal catalyst in a flow of a hydrogen gas under a pressure of from 20 to 200 kPa (absolute pressure).

The hydrogen gas may be used in the form of a single hydrogen gas or a mixed gas of a hydrogen gas and an inert gas, and is preferably used in the form of a single hydrogen gas. When using the mixed gas of a hydrogen gas and an inert gas, the concentration of the hydrogen gas therein is preferably 45% or more, more preferably 60% or more, and still more preferably 80% or more. As the inert gas, preferred are a nitrogen gas, an argon gas and a helium gas, and more preferred is a nitrogen gas.

The phrase "in a flow of a hydrogen gas" as used herein means such a condition that a hydrogen-containing gas is continuously or intermittently introduced into the reaction mixture, and a surplus of the hydrogen gas which is unconsumed in the reaction and/or a gas containing a water vapor produced during the reaction is continuously or intermittently discharged from the reaction mixture and in turn from a reaction apparatus.

The reaction temperature used in the dehydration and hydrogenation reaction is preferably from 70 to 300°C, more preferably from 90 to 200°C, still more preferably from 100 to 170°C, and especially preferably from 130 to 160°C from the viewpoints of completing the reaction for a short period of time and preventing occurrence of polymerization or decomposition of the raw material and intermediate product to enhance a yield of the aimed product.

In the dehydration and hydrogenation reaction, the compound represented by the following general formula (6) which is formed by reducing a carbonyl group of the aimed compound (2) (hereinafter occasionally referred to merely as a "compound (6)") may be produced as a by-product.

Therefore, in order to suppress production of the compound (6) and enhance a yield of the aimed compound, the reaction is preferably carried out in the pressure range of from reduced pressure to low applied pressure. The reaction pressure may vary depending upon the reaction temperature, and is preferably in the range of from 20 to 200 kPa (absolute pressure), more preferably from 50 to 150 kPa (absolute pressure), still more preferably from 70 to 120 kPa (absolute pressure), and especially preferably an atmospheric pressure. 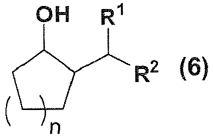 wherein n, R¹ and R² are the same as defined above.

In the process of the present invention, water is produced in the dehydration and hydrogenation reaction and contained in the reaction system. By flowing a hydrogen gas through the reaction system, it is possible to discharge water produced during the reaction out of the reaction system efficiently. Further, by conducting the reaction in a flow of a hydrogen gas while discharging water produced during the reaction out of the reaction system, it is considered that the dehydration and hydrogenation reaction is allowed to proceed even under almost normal pressures such as 200 kPa (absolute pressure) or less, even though the reaction is not carried out under such a high pressure condition (1.08 MPa (absolute pressure)) as described in Examples of Patent Document 4 (JP 61-5038A), so that production of the compound (6) as a by-product can be suppressed owing to such a low pressure.

The amount of the hydrogen gas introduced into the reaction system is preferably from 1 to 1000 N-mL/h, more preferably from 5 to 500 N-mL/h, and still more preferably from 10 to 100 N-mL/h per 1 g of the compound (1) charged from the viewpoints of a rate of consumption of hydrogen during the reaction and a good economy. Meanwhile, in the case where the amount of hydrogen introduced into the reaction system is less than the amount of hydrogen consumed during the reaction, there may occur the case where the gas discharged from the reaction system contains no hydrogen.

### (Reaction Apparatus)

The reaction apparatus used in the process of the present invention is not particularly limited as long as with a progress of the reaction, a surplus of the hydrogen gas and/or the water vapor produced can be discharged out of the reaction system while introducing the hydrogen gas into the reaction system, and further the other reaction conditions defined in the present invention can be applied thereto. The reaction apparatus may be either a batch-type reaction apparatus or a continuous-tube-type reaction apparatus. From the viewpoint of low costs for apparatuses, the batch-type reaction apparatus is preferably used.

### <Solvent>

The process of the present invention may be carried out either in the presence of a solvent or under a solvent-free condition. The process of the present invention is advantageously carried out under a solvent-free condition from the viewpoints of a good productivity and economy.

The solvent used in the process of the present invention is not particularly limited, and may be an inert organic solvent. Examples of the inert organic solvent include alcohols such as methanol, ethanol, propanol, isopropanol, isobutanol, tert-butanol, n-butanol, 2-butanol, isopentanol, pentanol, hexanol, 2-ethyl butanol, heptanol, 2-heptanol, octanol, 2-octanol, cyclopentanol, cyclohexanol, ethylene glycol, propylene glycol, ethylene glycol monoethyl ether, ethylene glycol monopropyl ether, propylene glycol monoethyl ether, diethylene glycol, diethylene glycol monomethyl ether, benzyl alcohol and phenyl ethanol; ketones such as methyl ethyl ketone, methyl isopropyl ketone, methyl propyl ketone, methyl isobutyl ketone, methyl n-butyl ketone, methyl n-amyl ketone, methyl isoamyl ketone, ethyl butyl ketone, methyl n-hexyl ketone, dipropyl ketone, diisobutyl ketone, cyclopentanone and cyclohexanone; ethers such as isopropyl ether, n-butyl ether, 1,4-dioxane, isoamyl ether, n-hexyl ether, tetrahydropyran 2-methyl furan, diethylene glycol diethyl ether, methyl phenyl ether and ethyl phenyl ether; esters such as n-methyl formate, n-propyl formate, n-butyl formate, methyl acetate, isopropyl acetate, n-butyl acetate, n-amyl acetate, n-hexyl acetate, cyclohexyl acetate, ethyl propionate, n-butyl propionate, methyl butyrate, n-butyl butyrate, methyl isovalerate, ethyl lactate, methyl benzoate, propyl benzoate, dimethyl phthalate, diethyl oxalate, dimethyl succinate, dimethyl glutarate and dimethyl adipate; and hydrocarbons such as n-hexane, n-octane, n-decane, ligroin, cyclohexane, benzene, toluene, xylene, ethyl benzene, isopropyl benzene, amyl benzene, t-butyl benzene, p-cymene, tetralin and decalin.

These solvents may be used alone or in combination of any two or more thereof.

The amount of the solvent used in the process of the present invention is preferably from 0.1 to 5 times by mass and more preferably from 0.3 to 2 times by mass on the basis of the compound (1) as the raw material.

The compound (2) obtained in first step of the process of the invention is further subjected to Baeyer-Villiger oxidation reaction using a peracetic acid or the like as an oxidizing agent (i.e., such an oxidation reaction in which a ketone and a percarboxylic acid are reacted to insert an oxygen atom into a position adjacent to a carbonyl group of the ketone and thereby obtain a carboxylic acid ester), for example, as described in JP 9-104681A, thereby producing a lactone represented by the following general formula (7) (5-alkyl-5-alkanolide or 6-alkyl-6-alkanolide) which is useful as a perfume material. 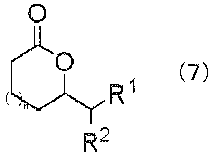 wherein n, R¹ and R² are the same as defined above.

The oxidation reaction step was carried out subsequently to the above dehydration and hydrogenation reaction step as a previous reaction step. The compound (2) may be subjected to purification treatment before subjected to the oxidation reaction step. However, the oxidation reaction step is preferably carried out after removing the catalyst only from the reaction mixture obtained in the dehydration and hydrogenation reaction step from the viewpoint of convenience and reaction control. When using the soluble noble metal catalyst in the previous reaction step, an absorbent may be added to remove the catalyst from the reaction mixture. Also, when using the soluble acid catalyst in the previous reaction step, a base may be added to neutralize the catalyst or an absorbent may be added to remove the catalyst from the reaction mixture. Further, when using the solid catalyst in the previous reaction step, the catalyst may be removed from the reaction mixture by solid-liquid separation procedure such as filtration and centrifugal separation.

The 2-alkylcycloalkanone as a substrate of the above oxidation reaction preferably has a ring structure corresponding to that represented by the general formula (7) in which n is 1 or 2, i.e., has a cyclopentanone ring or a cyclohexanone ring which can provide corresponding 5-alkyl-5-alkanolide or 6-alkyl-6-alkanolide.

The alkyl group as R¹ or R² in the 2-alkylcycloalkanone is preferably hydrogen or a linear or branched alkyl group having 1 to 8 carbon atoms. It is especially preferred that one of R¹ and R² be hydrogen and the other of R¹ and R² be an alkyl group having 4 to 7 carbon atoms.

As the oxidizing agent, there may be used various kinds of percarboxylic acids. From the viewpoints of a high reactivity and a facilitated purification step, metachloroperbenzoic acid and peracetic acid can be suitably used as the oxidizing agent. In addition, in place of using the percarboxylic acid itself, a mixture of a carboxylic acid and hydrogen peroxide may also be used to subsequently generate the percarboxylic acid in the reaction system.

The amount of the percarboxylic acid used in the oxidation reaction is not particularly limited, and is preferably from 0.9 to 2.0 mol per 1 mol of the 2-alkylcycloalkanone as the substrate.

The oxidation reaction step may be carried out without using any solvent. However, from the viewpoints of easiness of mixing and control of reaction rate, the solvent is preferably used in the oxidation reaction step. As the solvent, there may be used ordinary solvents except for ketones which are susceptible to oxidation reaction. When using a solvent in the dehydration and hydrogenation reaction step as the previous reaction step, the solvent may be used as such in the subsequent oxidation reaction step. When using the hydrogen peroxide and carboxylic acid, these compounds may be reacted in a two-phase system including a water phase and an oil phase.

The oxidation reaction temperature is preferably from 10 to 90°C and more preferably from 40 to 80°C from the viewpoint of obtaining the reaction product having a high purity.

After completion of the oxidation reaction, the resulting reaction product is subjected to purification treatment to thereby obtain a lactone useful for flavors, perfume materials for cosmetics, etc.

### EXAMPLES

In the following Examples and Comparative Examples, the term "%" represents "% by mass" unless otherwise specified. In addition, all of the reaction pressures used hereinafter represent an atmospheric pressure (101 kPa; absolute pressure).

The quantitative determination of respective reaction products was carried out by gas chromatography (GC) ("6890N" with a column "DB-1" (30 m x 0.25 mm x 0.25 µm) available from Agilent Technology Corp.; oven: 100°C → 5°C/min → 210°C → 20°C/min → 280°C (held for 4.5 min) (total 30 min); carrier: He; flow rate: 1.6 mL/min; inlet temperature: 200°C; detector (FID) temperature^{:} 280°C; amount charged: 1 µL; split: 100:1) according to an internal standard method (internal standard: undecane (purity: 99%; available from Nacalai Tesque, Inc.)).

### SYNTHESIS EXAMPLE 1 (Synthesis of 2-(1-hydroxypentyl)-cyclopentanone)

A 6 m³ reaction vessel equipped with a dropping tank was charged with 2241 kg (26.6 kmol) of cyclopentanone, 1007 kg of water and 11 kg of 48% NaOH. The contents of the reaction vessel were cooled to 15°C while stirring, and then 985 kg (11.4 kmol) of valeraldehyde were added dropwise thereto at the same temperature over 5 h, followed by stirring the resulting mixture for 1 h. After completion of the reaction, the resulting reaction mixture was neutralized, and an excess amount of cyclopentanone was recovered by distillation. As a result, it was confirmed that 1868 kg of the final reaction product obtained from an organic layer of the reaction mixture contained 1706 kg of 2-(1-hydroxypentyl)-cyclopentanone.

### EXAMPLE 1

### A 200 mL three-necked separable flask (made of glass) equipped with a dehydration tube was charged with 93.5 g (0.51 mol) of

2-(1-hydroxypentyl)-cyclopentanone (purity: 91%) obtained in Synthesis Example 1, 1.93 g of a phosphoric acid-supporting activated carbon (H₃PO₄/C; available from Taihei Chemical Industrial Co., Ltd.; powder, 22.1% hydrous product; phosphorus content: 0.012 (obtained by elemental analysis)), and 7.42 g (dry weight: 4.35 g) of 5% Pd/C (available from Evonik Degussa Japan Co., Ltd.; powder, 58.6% hydrous product). The contents of the flask were heated to 140°C while mixing under a pressure of 101 kPa (absolute pressure) while flowing a hydrogen gas (blowing the hydrogen gas into the reaction solution at a flow rate of 12 N-mL/h per 1 g of a pure content of 2-(1-hydroxypentyl)-cyclopentanone as the raw material). After reaching 140°C, the reaction solution was reacted for 20 h. After completion of the reaction, the final reaction product was subjected to quantitative determination by GC. As a result, it was confirmed that 0.50 mol of 2-pentyl-2-cyclopentanone was produced with a yield of 98%. Meanwhile, no 2-pentylcyclopentanol as a carbonyl group-reduced by-product was detected.

### REFERENCE COMPARATIVE EXAMPLE 1

A 200 mL SUS autoclave was charged with 100 g of diacetone alcohol, 1 g of 5% Pd/C and 1 g of a strong acid ion exchange resin "AMBERLYST (registered trademark) 15" available from Rohm & Haas Co., and the contents of the autoclave were reacted at a temperature of 120°C under a hydrogen pressure of 1.08 MPa (absolute pressure). After the elapse of about 80 min, absorption of a predetermined amount of hydrogen was recognized. As a result of analyzing the reaction solution by gas chromatography, it was confirmed that the conversion rate of diacetone alcohol was 94.9%, and the selectivity to methyl isobutyl ketone as the aimed product was 92.8%, whereas the selectivity to methyl isobutyl carbinol as the carbonyl group-reduced by-product was 2.6%.

### EXAMPLE 2

A 1 L four-necked flask was charged with 543 g (3.45 mol) of pentylcyclopentanone obtained by the same method as in Example 1, and then the content of the flask was subjected to distillation using a distillation vessel equipped with a 8 cm Vigreux column (vessel inside temperature: 89°C (finally reached); vessel top temperature: 80°C; reduced pressure: 500 Pa, 3.8 Torr), thereby obtaining a fraction having a purity of 97.9%.

A 2 L four-necked flask equipped with a cooling tube, a thermometer and a dropping funnel was charged with 200 g of pentylcyclopentanone obtained above, 800 g of toluene, 1.0 eq. of acetic acid (based on a pure content of the raw material) and 16 g of water, and the contents of the flask were heated to 65°C. After reaching 65°C, 173 g (1.4 eq.) of 35% aqueous hydrogen peroxide were added dropwise to the flask over 1.5 h. After completion of the dropping, the contents of the flask were heated to 70°C and aged as such for 48 h.

After completion of the aging, the stirring was stopped, and the contents of the flask were allowed to stand and separated into upper and lower layers to withdraw and remove the lower layer therefrom. The upper layer was washed with 165 g of a 10% sodium sulfite aqueous solution twice to decompose a peracid remaining in the upper layer and remove the decomposed product therefrom, and further washed with 100 g of a 10% sodium sulfate aqueous solution twice.

The upper layer thus washed was dried with magnesium sulfate and filtered, and then the solvent was removed by distillation therefrom using an evaporator (finally reached 70°C /2.7 kPa (20 Torr)). It was confirmed that the conversion rate of the raw material was 92%, and the selectivity of the δ-decalactone in a converted material was 86%. After removal of the solvent by distillation, a high-boiling component was removed from the reaction solution by simple distillation, and the reaction solution was subjected to rectification and purification treatment using a 20-stage rectifying column, thereby obtaining δ-decalactone having a purity of 99%.

### INDUSTRIAL APPLICABILITY

In accordance with the present invention, there is provided process for producing lactones which are useful as a perfume material for perfumes/cosmetics, flavors or the like efficiently and at low costs.

## Claims

1. A process for producing a lactone, comprising the steps of:
producing a 2-alkylcycloalkanone represented by the following general formula (2) by a process comprising the step of subjecting a 2-(1-hydroxyalkyl)-cycloalkanone represented by the following general formula (1) to dehydration and hydrogenation reaction in a flow of a hydrogen gas under a pressure of from 20 to 200 kPa (absolute pressure) in the presence of an acid and a platinum group metal catalyst; and then
subjecting the 2-alkylcycloalkanone to oxidation reaction using a percarboxylic acid: 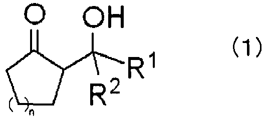 wherein n is an integer of 1 or 2; and R¹ and R² are each independently a hydrogen atom or an alkyl group having 1 to 8 carbon atoms with the proviso that R¹ and R² may form a cyclopentane ring or a cyclohexane ring through a carbon atom adjacent thereto, wherein hydrogen atom(s) bonded to carbon atoms constituting said cyclopentane ring or said cyclohexane ring may be substituted with a hydrocarbon group having 1 to 5 carbon atoms; and 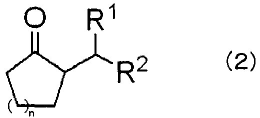 wherein n, R¹ and R² are the same as defined above.

2. The process for producing a lactone according to claim 1, wherein the acid has a first acid dissociation constant (pKa) of 0 or more as measured at 25°C.

3. The process for producing a lactone according to claim 1 or 2, wherein the acid is a phosphoric acid-supporting activated carbon.

4. The process for producing a lactone according to claim 1, wherein the acid is a solid acid.

5. The process for producing a lactone according to claim 4, wherein an amount (mmol/g) of acid sites of the solid acid from which NH₃ is desorbed at a temperature of from 100 to 250°C is larger than an amount (mmol/g) of acid sites of the solid acid from which NH₃ is desorbed at a temperature higher than 250°C, as measured by an ammonia temperature-programmed desorption (TPD) method.

6. The process for producing a lactone according to any one of claims 1 to 5, wherein the platinum group metal catalyst is a Pd- and/or Pt-containing catalyst.

7. The process for producing a lactone according to any one of claims 1 to 6, wherein the reaction is carried out under a pressure of from 50 to 150 kPa (absolute pressure).

## Patentansprüche

1. Verfahren zur Herstellung eines Lactons, umfassend die Schritte:
Herstellung eines 2-Alkylcycloalkanons der nachstehenden allgemeinen Formel (2) durch ein Verfahren, umfassend einen Schritt, in dem ein 2-(1-Hydroxyalkyl)-cycloalkanon der nachstehenden allgemeinen Formel (1) einer Dehydrierungs- und Hydrierungsreaktion in einem Wasserstoffgasstrom unter einem Druck von 20 bis 200 kPa (absoluter Druck) in Gegenwart einer Säure und einem Platingruppen-Metallkatalysator unterworfen wird; und dann
Unterwerfen des 2-Alkylcycloalkanons einer Oxidationsreaktion unter Verwendung einer Percarbonsäure: 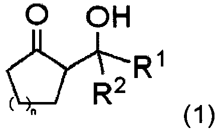 worin n eine ganze Zahl von 1 oder 2 ist und R¹ und R² jeweils unabhängig ein Wasserstoffatom oder eine Alkylgruppe mit 1 bis 8 Kohlenstoffatomen sind, mit der Massgabe, dass R¹ und R² durch ein hierzu benachbartes Kohlenstoffatom einen Cyclopentanring oder einen Cyclohexanring bilden können, wobei (ein) Wasserstoffatom(e), das/die an die Kohlenstoffatome gebunden ist/sind, die den Cyclopentanring oder den Cyclohexanring bilden, mit einer Kohlenwasserstoffgruppe mit 1 bis 5 Kohlenstoffatomen substituiert sein kann; und 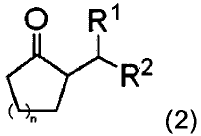 worin n, R¹ und R² die gleichen sind wie oben definiert.

2. Verfahren zur Herstellung eines Lactons gemäss Anspruch 1, wobei die Säure eine erste Säuredissoziationskonstante (pKa) von 0 oder mehr, gemessen bei 25°C, aufweist.

3. Verfahren zur Herstellung eines Lactons gemäss Anspruch 1 oder 2, wobei die Säure eine Phosphorsäure tragende Aktivkohle ist.

4. Verfahren zur Herstellung eines Lactons gemäss Anspruch 1, wobei die Säure eine feste Säure ist.

5. Verfahren zur Herstellung eines Lactons gemäss Anspruch 4, wobei die Menge (mmol/g) der Säurestellen der festen Säure, von der NH₃ bei einer Temperatur von 100 bis 250°C desorbiert ist, grösser ist als die Menge (mmol/g) der Säurestellen der festen Säure, von der NH₃ bei einer Temperatur von höher als 250°C desorbiert ist, wie durch ein Verfahren mit einer Temperatur-programmierten Ammoniak-Desorption (TPD) gemessen.

6. Verfahren zur Herstellung eines Lactons gemäss irgendeinem der Ansprüche 1 bis 5, wobei der Platingruppen-Metallkatalysator ein Pd- und/oder Pthaltiger Katalysator ist.

7. Verfahren zur Herstellung eines Lactons gemäss irgendeinem der Ansprüche 1 bis 6, wobei die Reaktion unter einem Druck von 50 bis 150 kPa (absoluter Druck) durchgeführt wird.

## Revendications

1. Procédé de production d'une lactone, comprenant les étapes de :
production d'une 2-alkylcycloalcanone représentée par la formule générale (2) suivante par un procédé comprenant l'étape de soumission d'une 2-(1-hydroxyalkyl)-cycloalcanone représentée par la formule générale (1) suivante à une réaction de déshydratation et d'hydrogénation dans un flux d'hydrogène gazeux sous une pression de 20 à 200 kPa (pression absolue) en présence d'un acide et d'un catalyseur à base d'un métal du groupe du platine ; et ensuite soumission de la 2-alkylcycloalcanone à une réaction d'oxydation à l'aide d'un acide percarboxylique : 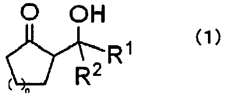 dans lequel n est un nombre entier de 1 ou 2 ; et R¹ et R² sont chacun indépendamment un atome d'hydrogène ou un groupe alkyle comportant 1 à 8 atomes de carbone à condition que R¹ et R² puissent former un cycle cyclopentane ou un cycle cyclohexane à travers un atome de carbone adjacent à ceux-ci, dans lequel le ou les atomes d'hydrogène liés aux atomes de carbone constituant ledit cycle cyclopentane ou ledit cycle cyclohexane peuvent être substitués par un groupe hydrocarboné comportant 1 à 5 atomes de carbone ; et 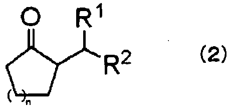 dans lequel n, R¹ et R² sont tels que définis ci-dessus.

2. Procédé de production d'une lactone selon la revendication 1, dans lequel l'acide a une première constante de dissociation d'acide (pKa) de 0 ou plus telle que mesurée à 25 °C.

3. Procédé de production d'une lactone selon la revendication 1 ou 2, dans lequel l'acide est du charbon actif supportant de l'acide phosphorique.

4. Procédé de production d'une lactone selon la revendication 1, dans lequel l'acide est un acide solide.

5. Procédé de production d'une lactone selon la revendication 4, dans lequel une quantité (mmol/g) de sites acides de l'acide solide duquel NH₃ est désorbé à une température de 100 à 250 °C est supérieure à une quantité (mmol/g) de sites acides de l'acide solide duquel NH₃ est désorbé à une température supérieure à 250 °C, telle que mesurée par un procédé de désorption d'ammoniaque à température programmée (TPD).

6. Procédé de production d'une lactone selon l'une quelconque des revendications 1 à 5, dans lequel le catalyseur à base d'un métal du groupe du platine est un catalyseur contenant du Pd et/ou du Pt.

7. Procédé de production d'une lactone selon l'une quelconque des revendications 1 à 6, dans lequel la réaction est réalisée sous une pression de 50 à 150 kPa (pression absolue).
